(19) Europäisches Patentamt
European Patent Office
Office européen des brevets

(11) Publication number: **0 034 238**
**B1**

(12) **EUROPEAN PATENT SPECIFICATION**

(45) Date of publication of patent specification: **17.04.85**

(51) Int. Cl.[4]: **C 07 F 5/02,** A 61 K 31/69

(21) Application number: **80810406.1**

(22) Date of filing: **19.12.80**

(54) **Pharmacologically active amine-carboxyboranes.**

(30) Priority: **21.12.79 US 106416**

(43) Date of publication of application:
**26.08.81 Bulletin 81/34**

(45) Publication of the grant of the patent:
**17.04.85 Bulletin 85/16**

(84) Designated Contracting States:
**AT BE CH DE FR GB IT LI NL**

(56) References cited:
**JOURNAL OF THE AMERICAN CHEMICAL SOCIETY, Vol. 102, No. 20, 24 September 1980, Gaston B.F. SPIELVOGEL et al. "Boron Analogues of the alpha-Amino Acids. Synthesis, X-ray Crystal Structure, and Biological Activity of Ammonia-Carboxyborane, the Boron Analogue of Glycine" pages 6343 to 6344**

**The file contains technical information submitted after the application was filed and not included in this specification**

(73) Proprietor: **Duke University**
**Office of Patent Administration 614 Chapel Drive Annex**
**Durham North Carolina 27706 (US)**

(72) Inventor: **Spielvogel, Bernard F.**
**P.O. Box 12223**
**Research Triangle Park N.C. 27709 (US)**
Inventor: **McPhail, Andrew T.**
**331 Smith Drive**
**Durham N.C. 27712 (US)**
Inventor: **Hall, Iris H.**
**328 Azalen Dr. Rt. 7**
**Chapel Hill N.C. 27514 (US)**

(74) Representative: **Mongrédien, André et al**
**c/o BREVATOME 25, rue de Ponthieu**
**F-75008 Paris (FR)**

(56) References cited:
**JOURNAL OF THE AMERICAN CHEMICAL SOCIETY, Vol. 98, No. 18, 1 September 1976 Gaston B.F. SPIELVOGEL et al. "Boron Analogues of Amino Acids. Synthesis and Biological Activity of Boron Analogues of Betaine" pages 5702 to 5703**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 034 238 B1

JOURNAL OF INORGANIC & NUCLEAR CHEMISTRY, Vol. 41, No. 7, 12 September 1979 Oxford B.F. SPIELVOGEL et al. "Synthesis of some Cyano-, Amido- and Carboxyborane Adducts of Amines and Diamines" pages 1223 to 1227

Chemical Abstracts vol. 63, no. 2, July 1965 Columbus, Ohio, USA J.C. CARTER et al. "The ammonia and alkylamine addition compounds of carbon monoxide borane" column 1466f-g

**Description**

Current research investigations for compounds having desirable biological activities and use in cancer chemotherapy treatments reveal the need for compounds with minimal toxicity and increased biological activity. The use of boron compounds in neutron capture therapy was suggested as early as 1936 by G. I. Locher, American Journal, Vol. 36, p.1 (1936). The rationale for this therapy, now quite familiar to boron chemists, rests upon the fact that $10_B$ and thermal neutrons in a nuclear reaction are capable of destroying cells. The only very important requirement for utilization of this approach is a large concentration differential of $10_B$ between the neoplasm and normal tissue so that only the tumor is destroyed. Included in the neutron-capture therapy approach is the concept, advanced by A. H. Soloway, *Progress In Boron Chemistry*, Vol. 1, The MacMillan Company, New York, 1964, Chapter 4, of preparing antimetabolites or other carcinostatic agents containing boron for a possible two-fold attack on a neoplasm. Thus, direct inhibition of tumor growth by the boron compound could be coupled with selective incorporation of the compound into the neoplasm with concomitant use of the neutron capture theory.

The use of boron compounds consisting of amine derivatives of carboxy borane for inhibiting tumor growth was suggested by B. F. Spielvogel J. of Inorg. and Nuclear Chem., vol. 41, pp. 123 (1979). However, none of the carboxyborane derivatives disclosed in this paper have a hydrogen on the amino nitrogen atom. As pointed out in the middle of the only text paragraph on page 1225 of this paper, new compounds 5 and 6 were only the second and third known examples of an amine carboxyborane, with trimethylamine carboxyborane being the only other known example. Accordingly, at the time of this paper, the only known amine carboxyboranes were ones in which no hydrogens were present on the amine nitrogen.

Summary of the invention

According to this invention, there are provided amine-carboxyboranes depicted by the chemical formula

$$R_1R_2NHH_2BC(O)OH$$

wherein $R_1$ and $R_2$ are selected from hydrogen, methyl, ethyl, propyl and n-butyl which exhibit significant antihyperlipidemic and anticancer activity.

Description of the preferred embodiments

The process of preparing the novel amine-carboxyboranes and their utilities are more fully illustrated in the following discussion and examples. The relative amounts of the reactants mentioned herein are in parts by weight and room temperature includes the temperature range of 20—30°C.

Example I

Ammonia-carboxyborane ($H_3N \cdot H_2BC(O)OH$) descriptive discussion—(synthesis, X-ray crystal structure, and biological activity of ammonia-carboxyborane, the boron analogue of glycine)

The boron analogue of glycine, ammonia-carboxyborane ($H_3N \cdot BH_2CO_2H$), was prepared by an amine displacement reaction on $Me_3N \cdot BH_2CO_2H$, and structurally characterized by single-crystal X-ray analysis; biological assays indicate that this compound is biologically active, in particular it has been shown to possess significant antihyperlimpidemic activity.

Glycine, $H_2NCH_2CO_2H$ or $H_3\overset{+}{N}CH_2CO_2^-$, may be considered the simplest alpha-amino acid and was the first to be discovered. We have now discovered the synthesis and characterization of its isoelectronic and isosteric (protonated) boron analogue, ammonia-carboxyborane, $H_3N \cdot BH_2CO_2H$ referred to in Table 1 as Compound 1. This compound may be viewed as the parent of a novel class of boron analogues of the command alpha-amino acids. Although we have previously reported the synthesis of other amine-carboxyboranes, e.g. $Me_3N \cdot BH_2CO_2H$, the boron analogue of betaine in the *Journal of America Chemical Society*, Vol. 98, p. 5703, 1976, all previous examples did not contain hydrogen bonded directly to nitrogen. An extremely important feature of the present example, like its glycine counterpart, is its potential to form peptide linkages and to be incorporated into proteins. The structure of Compound 1 has been unequivocally established by single-crystal X-ray analysis and its stability in several media investigated. Preliminary testing of Compound 1 in animal model studies has demonstrated biological activity, in particular, significant antihyperlipidemic activity.

Preparation of ammonia-carboxyborane was achieved by an amine exchange reaction. One part of $Me_3N \cdot BH_2CO_2H$ was allowed to react with 10 parts liquid $NH_3$ in an evacuated stainless steel cylinder at room temperature for 3 weeks after which time excess ammonia was allowed to evaporate off and removed finally by pumping out. The residue was transferred to a flask with the aid of dry $CHCl_3$, refluxed for 3—4 hours, filtered hot, and washed with hot $CHCl_3$ to give $H_3N \cdot BH_2CO_2H$ as an insoluble residue. This procedure led to a yield of crude product in the 50—55% range which remained essentially constant even when the time of reaction was increased from 3 to 6 weeks; a reaction period of 2 weeks gave $H_3N \cdot BH_2CO_2H$ in 30% yield. When the crude product is freshly crystallized from cold water it melts at 116°C but this is lowered, with no observable change in spectral characteristics, after storage of the product in screw-capped vials over a period of time in normal laboratory conditions. I.r. (KBr in $cm^{-1}$): nu (NH), a broad envelope with peaks at 3330 s, 3250 s, 3200 b, s; nu (OH) 3000 b; nu (NH. . .0) 2780 b, s, 2650 s; nu

(BH) 2400 s, 2340 sh, 2250 sh; (delta+tau) (NH) 2050 w, 1840 b, w, 1760 b, w; nu (CO) 1640 s, delta (NH) 1650 s. The alphabetical symbols as used above and throughout the specification are defined as follows: s—absorption; b—broad absorption; sh—shoulder absorption and w—weak absorption.

Single-crystal X-ray analysis established the structure of ammonia-carboxyborane unequivocally. Monoclinic crystals of ammonia-carboxyborane belong to space group $P2_1/c$, with $a$=4.859 (2) Å, $b$=5.291 (2) A, $c$=15.523 (7) Å beta=108.42 (3)°, $U$=378.6 Å$^3$, $Z$=4, $d_c$=1.313 g cm$^{-3}$. The structure was solved by direct methods using the MULTAN program package as described G. Germain et al, *Acta Crystallogr.*, Section A, Vol. 27, p. 368. Full-matrix least-squares refinement of atomic positional and thermal (anisotropic B, C, N, O; isotropic H) parameters converged to an $R^4$ value of 0.052 over 598 statistically significant [$I$ greater than 2.0 sigma ($I$)] reflections measured on an Enraf-Nonius CAD-3 automated diffractometer (Ni-filtered Cu-K alpha radiation, lambda=1.5418 Å theta-2 theta scans). Although molecules of $H_3N \cdot H_2BC(O)OH$ exist in the solid state in a form typical of optically inactive or racemic carboxylic acids, *i.e.* as centrosymmetric dimers, they have a slightly longer, and thus weaker, O—H. . .O hydrogen bonded distance [O. . .O 2.668 (2) Å in $H_3N \cdot H_2BC(O)OH$ *vs. ca.* 2.64 Å in simple acids]. Dimers of $H_3N \cdot H_2BC(O)OH$ are further associated *via* interdimer N—H. . .O hydrogen bonds [N. . . O 2.981 and 3.157 Å] involving two of the amino hydrogen atoms, a class of relatively strong intermolecular interactions not available to simple carboxylic acids, and a feature which must be partly responsible for the elevated melting point of $H_3N \cdot H_2BC(O)OH$ compared to propionic acid (−20.8 C).

Hydrolysis of $H_3N \cdot H_2BC(O)OH$ occurs very slowly as manifested by the observation that a 0.118 $M$ solution thereof in water underwent only trace decomposition in 3 hours. Very slow decomposition of $H_3N \cdot H_2BC(O)OH$ also took place in alkali *e.g.* a 0.126 $M$ solution thereof in 1$N$ NaOH underwent only 0.33% decomposition in 3 hours, with tapering off after that time, only trace amounts of gas being evolved in the following 3 days. In contrast, $H_3N \cdot H_2BC(O)OH$ is readily hydrolyzed in acid, *e.g.* a 2.23:1 mole ratio of gas to $H_3N \cdot H_2BC(O)OH$ was evolved after 3.5 hours from a 0.126 $M$ solution of $H_3N \cdot H_2BC(O)OH$ in 1$N$ HCl, and this increased to 2.27 after 20.5 hours by which time gas evolution was complete. Assuming that only hydrolysis of the B—H bonds occurs, a total of two moles of $H_2$ per mole of $H_3N \cdot H_2BC(O)OH$ would be anticipated. However, i.r. analysis of the evolved gas showed that it contained CO, a fact which may be indicative of the involvement of a carbonyl intermediate $[H_3N \cdot BH_2CO]^+$ which could undergo subsequent hydrolysis to yield CO and $H_2$. That compound ammonia-carboxyborane ($H_3N \cdot H_2BC(O)OH$) is reasonable thermally stable was demonstrated by heating 0.778 mmol $H_3N \cdot H_2BC(O)OH$ in an evacuated flask at 60°C for 8 hours when only 0.55 mole % was found to be decomposed.

Significant antitumor activity observed following the procedures described by C. Piantadosi et al in the *Journal Pharm. Sci.*, Vol. 58, p. 821 (1969). The control in this screen, 6-mercaptopurine, showed 99% inhibition. Additionally, significant antihyperlipidemic activities where serum cholestrol was assayed by means of the Lieberman-Burchard reaction. The control in this assay, clofibrate, which requires 300 mg/kg for significant antihyperlipidemic activity, showed 98% inhibition. In each of the tests evaluating the antitumor and antihyperlipidemic activities a dose of 20 mg/kg per day of $H_3N \cdot H_2BC(O)OH$ was administered to $CF_1$ male mice; the $LD_{50}$ is greater than 0.2 grams/kg. In the Ehrlich Ascites screen, inhibition of tumor growth was 76.5% for $H_3N \cdot H_2BC(O)OH$, while lowering of the serum cholesterol level was 44% after 9 days and 60% after 16 days.

Example II

Dimethylamine-carboxyborane ($(CH_3)_2NH \cdot H_2BC(O)OH$) descriptive discussion—(synthesis, X-ray crystal structure, and antitumor activity of dimethylamine-carboxyborane the boron analogue of N,N-dimethylglycine)

The biologically active boron analogue of *N,N* - dimethylglycine, $Me_2NH \cdot BH_2COOH$, was prepared by an amine displacement reaction on $Me_3N \cdot BH_2COOH$ and has been structurally characterized by single-crystal *X*-ray analysis.

Preparation of dimethylamine-carboxyborane was achieved by the interaction of 10 parts dimethylamine with 1 part of trimethylamine-carboxyborane ($(CH_3)_3N \cdot BH_2C(O)OH$) in a steel bomb at room temperature for a period of 15 days. Unreacted trimethylamine-carboxyborane was removed by dissolution in $CHCl_3$ at room temperature, leaving dimethylamine-carboxyborane which could be recrystallized from hot $CHCl_3$. Yields of dimethylamine-carboxyborane up to 80% can be obtained by extended reaction. Satisfactory analytical data were obtained; m.p. 105 C (decomp.); nu (Nujol) NH 3220 (sh); OH 3160; BH 2370 (s), 2290 (m); CO 1640 (s) cm$^{-1}$; delta ($NMe_2$): 2.45 ppm in $D_2O$, taking delta (HOD)=4.70 ppm as reference. The structure was confirmed unequivocally by single-crystal *X*-ray analysis.

Crystal data: $C_3H_{10}BNO_2$, $M$=102.93, monoclinic, space group $P2_1/c$, $a$=10.548 (5), $b$=6.600 (3), $c$=9.395 (5) Å, beta=90.98 (5)°, $U$=654.0 Å$^3$, $Z$=4, $D_c$=1.045 g cm$^{-3}$. Intensity data to theta 67° were recorded on an Enraf-Nonius CAD-3 automated diffractometer (Ni-filtered Cu-$K_{alpha}$ radiation, lambda=1.5418 Å; theta-2 theta scans). The structure was solved by direct methods. Atomic positional and thermal parameters (anisotropic B, C, N, O; isotropic H) were refined by full-matrix least-squares calculations to $R$ 0.066 over 885 statistically significant reflections. In the crystal the dimers are further associated through N—H. . .O hydrogen bonding (N. . . .O 2.867 Å).

Although dimethylamine-carboxyborane is stable in air, the m.p. loses its sharpness when it is kept in normal laboratory conditions for a long time; other spectroscopic properties remain the same. It is also

thermally quite stable; when heated in an evacuated flask at 60°C for 8 hours, it partly sublimed with only *ca.* 0.7% evolution of $H_2$. The considerable hydrolytic stability of dimethylamine-carboxyborane was demonstrated by the fact that a *ca.* 0.05*M* solution thereof in $H_2O$ produced only *ca.* 1.5% of the theoretical amount of $H_2$ after 2 days (assuming complete decomposition to $H_2$), *ca.* 2.0% in one week, and 4.4% in three weeks. Moreover, dimethylamine-carboxyborane was found to be stable in strong base as no measureable amount of $H_2$ was evolved in 7 days from a *ca.* 0.06*M* solution thereof in 1*N* NaOH. On the other hand, however, dimethylamine-carboxyborane is readily hydrolyzed by 1*N* HCl as indicated by the liberation of more than the theoretical amount of gas within 2 days; the i.r. spectrum of the generated gas showed that carbon monoxide (CO) had been liberated in addition to $H_2$. Evolution of CO in this case may be indicative of a second reaction pathway with acid according to equation (1). The intermediate, $[Me_2NH \cdot BH_2CO]^+$,

$$Me_2NH \cdot BH_2COOH + H^+ \rightarrow [Me_2NH \cdot BH_2CO]^+ + H_2O \qquad (1)$$

could undergo subsequent hydrolysis to give CO and $H_2$ in a manner similar to the reported hydrolysis of $BH_3CO$.

In the Ehrlich Ascites antitumour screen, dosages of 33.3 mg/kg per day of dimethylamine-carboxyborane into $CF_1$ male mice resulted in inhibition of tumour growth of 94.6%; the $LD_{50}$ for this compound is in excess of 200 mg/kg.

Example III

Methylamine-carboxyborane ($CH_3NH_2 \cdot H_2BC(O)OH$) descriptive discussion—(synthesis, X-ray crystal structure, and biological activity of methylamine-carboxyborane, the boron analogue of sarcosine)

The boron analogue of sarcosine (*N*-methylglycine), $MeNH_2 \cdot BH_2COOH$, was prepared by an amine displacement reaction on $Me_3N \cdot BH_2COOH$, and structurally characterized by single-crystal X-ray analysis; biological assays indicate that this compound has significant antitumor and antihyperlipidemic as well as moderate antiinflammatory activity.

The existence of a class of isoelectronic and isosteric boron containing alpha-amino acid analogues possessing biological activity would be expected to have considerable impact on many scientific and medical fields. Directed towards our goal of synthesizing boron analogues of the common alpha-amino acids, we have discovered the synthesis, X-ray crystal structure, and biological activity of methylamine-carboxyborane, $MeNH_2 \cdot BH_2COOH$, the boron analogue of sarcosine (*N*-methylglycine).

Preparation of methylamine-carboxyborane was achieved by the interaction of 10 parts of methylamine with 1 part $Me_3N \cdot BH_2COOH$ in a stainless steel bomb at room temperature for 15 days. The unreacted trimethylamine-carboxyborane was removed by dissolution in hot $CHCl_3$, leaving methylamine-carboxyborane which could be recrystallized from cold $H_2O$; the yield before recrystallization was more than 95%. Satisfactory analytical data were obtained: m.p. 108—9C (decomposed); i.r. (KBr in $cm^{-1}$) 3270 s and 3190 s $nu_{NH}$, 3040 s $nu_{OH}$, 1640 s $nu_{CO}$, 1600 s $delta_{NH}$; $^1H$ NMR ($D_2O$) delta 2.3 ($CH_3N$) 4.7 (s HCO); mass spectrum *m/e* 89 ($M^+$, $C_2H_8{}^{11}BNO_2$).

Single-crystal X-ray analysis established the structure of methylamine-carboxyborane unequivocally. Monoclinic crystals of methylamine-carboxyborane belong to space group $P2_1/c$, $a$=5.125 (2), $b$=5.509 (3), $c$=17.289 (8) Å, beta=99.91 (5), $U$=481 Å, $Z$=4, $d_c$=1.228 g $cm^{-3}$. The structure was solved by direct methods disclosed in the article by G. Germain et al cited above. Full-matrix least-squares refinement of atomic positional and thermal (anisotropic B, C, N, O; isotropic H) parameters converged to an $R$ value of 0.039 over 690 statistically significant (I greater than 2.0 sigma (I)) reflections measured on an Enraf-Nonius CAD-3 automated diffractometer (Ni-filtered Cu-*K* alpha radiation, lambda=1.5418 Å; theta-2 theta scans). The conformation and molecular dimensions of the centrosymmetric dimers were found in the solid state. As with trimethylamine-carboxyborane the intradimer hydrogen bonded O. . .O separation at 2.688 Å in methylamine-carboxyborane is slightly longer than the corresponding value of *ca* 2.64 Å which is found in simple carboxylic acids. The dimers are further associated in the crystal by weak N—H. . .O hydrogen bonds (N. . .O 3.101 and 3.102 Å).

Compound methylamine-carboxyborane proved to be fairly stable in air as evidenced by the fact that although prolonged exposure to normal laboratory conditions resulted in a slight lowering of the m.p., the i.r. spectrum remained unchanged. In water, slow decomposition of methylamine-carboxyborane occurred; typically, a 0.0443*M* solution thereof in water produced 1/4% of the theoretical amount of $H_2$ after 3 hours (assuming complete decomposition to $H_2$), 1.7% in 21 hours, 2.4% in 7 days, and 2.9% in 14 days. Slow decomposition of methylamine-carboxyborane also took place in 1*N* NaOH solution; a 0.0423*M* solution thereof in 1*N* NaOH liberated no gas in 3 hours, 0.25% of H in 18 hours, and 0.85% in 7 days. In contrast to its relative stability in water and NaOH solution, methylamine-carboxyborane is sensitive to acid. Rapid decomposition of methylamine-carboxyborane in 1*N* HCl was accompanied by the generation of greater than the theoretical amount of $H_2$ in 24 hours; the i.r. spectrum of the generated gas showed that carbon monoxide (CO) was liberated in addition to $H_2$. Evolution of carbon monoxide may be indicative of a reaction pathway with acid whereby formation of the intermediate $[MeNH_2 \cdot BH_2CO]^+$ is followed by its hydrolysis to yield CO and $H_2$ in a manner similar to the reported hydrolysis of $BH_3CO$.

Methylamine-carboxyborane is thermally fairly stable; on heating a sample thereof in an evacuated flask at 60°C for 8 hours, only 0.6 mole % evolution of $H_2$ was observed.

Significant antitumor and antihyperlipidemic activities were demonstrated when dosages of 20 mg/kg per day of methylamine-carboxyborane were administered to $CF_1$ male mice; the $LD_{50}$[10] is greater than 1 g/kg. In the Ehrlich Ascites screen, inhibition of tumor growth was 94% for methylamine-carboxyborane, while lowering of the serum cholesterol level was 34% after 9 days and 33% after 16 days. Moderate antiinflammatory activity (46% inhibition by a dosage of 20 mg/kg of methylamine-carboxyborane) was also indicated.

TABLE 1

| Compound | Ehrlich ascites % inhibition | Antiinflammatory % inhibition | Serum cholesterol % inhibition | $LD_{50}$ mg/kg |
|---|---|---|---|---|
| 1. $NH_3BH_2C(O)OH$ | 76.5 | 16 | 60 | greater than 200 |
| 2. $(CH_3)_2NHBH_2C(O)OH$ | 94.6 | 9 | 34 | greater than 200 |
| 3. $CH_3NH_2BH_2C(O)OH$ | 93.7 | 46 | — | greater than 1000 |
| Dosage | 20 mg/kg/day | 20 mg/kg×2 | 20 mg/kg/day | |

**Claims**

1. The compounds of the formula $R_1R_2NHBH_2C(O)OH$ wherein $R_1$ and $R_2$ are selected from hydrogen, methyl, ethyl, propyl and n-butyl.

2. The compound of Claim 1 wherein $R_1$ and $R_2$ are hydrogen.

3. The compound of Claim 1 wherein $R_1$ and $R_2$ are methyl.

4. The compound of Claim 1 wherein $R_1$ is hydrogen and $R_2$ is methyl.

5. The process for preparing the compounds of Claim 1 wherein 1 part of trimethylamine-carboxyborane is reacted with 10 parts of a compound having the formula $R_1R_2NH$, wherein $R_1$ and $R_2$ are as defined in claim 1, at a temperature ranging from 20—30°C for a period of 15 days.

6. The process of Claim 5 wherein $R_1$ and $R_2$ are hydrogen.

7. The process of Claim 5 wherein $R_1$ and $R_2$ are methyl.

8. The process of Claim 5 wherein $R_1$ is hydrogen and $R_2$ is methyl.

9. Pharmaceutical composition for use in the chemotherapeutical treatment of cancerous tissue which comprises an amine carboxyborane having the formula, $R_1R_2NHBH_2C(O)OH$ wherein $R_1$ and $R_2$ are selected from hydrogen, methyl, ethyl, propyl, and n-butyl.

10. Pharmaceutical composition of Claim 9 wherein $R_1$ and $R_2$ are hydrogen.

11. Pharmaceutical composition of Claim 9 wherein $R_1$ and $R_2$ are methyl.

12. Pharmaceutical composition of Claim 9 wherein $R_1$ is methyl and $R_2$ is hydrogen.

13. Pharmaceutical composition having an antihyperlipidemic activity which comprises a compound having the formula, $R_1R_2NHBH_2C(O)OH$ wherein $R_1$ and $R_2$ are selected from hydrogen, methyl, ethyl, propyl, and n-butyl.

14. Pharmaceutical composition of Claim 13 wherein $R_1$ and $R_2$ are hydrogen.

15. Pharmaceutical composition of Claim 13 wherein $R_1$ and $R_2$ are methyl.

16. Pharmaceutical composition of Claim 13 wherein $R_1$ is methyl and $R_2$ is hydrogen.

**Revendications**

1. Composés répondant à la formule $R_1R_2NHBH_2C(O)OH$ dans laquelle $R_1$ et $R_2$ sont choisis parmi l'hydrogène, le méthyle, l'éthyle, le propyle et le n-butyle.

2. Composé suivant la revendication 1, caractérisé en ce que $R_1$ et $R_2$ sont l'hydrogène.

3. Composé suivant la revendication 1, caractérisé en ce que $R_1$ et $R_2$ sont des méthyles.

4. Composé suivant la revendication 1, caractérisé en ce que $R_1$ est l'hydrogène et $R_2$ est le méthyle.

5. Procédé de préparation de composés suivant la revendication 1, caractérisé en ce qu'on fait réagir une partie de triméthylamine-carboxyborane avec dix parties d'un composé répondant à la formule $R_1R_2NH$, dans laquelle $R_1$ et $R_2$ sont tels que définis dans la revendications 1, à une température allant de 20 à 30°C, pendant une période de quinze jours.

6. Procédé suivant la revendication 5, caractérisé en ce que $R_1$ et $R_2$ sont l'hydrogène.

7. Procédé suivant la revendication 5, caractérisé en ce que $R_1$ et $R_2$ sont des méthyles.

8. Procédé suivant la revendication 5, caractérisé en ce que $R_1$ est l'hydrogène et $R_2$ est le méthyle.

9. Composition pharmaceutique pour l'utilisation dans le traitement chimiothérapique des tissus cancéreux, caractérisée en ce qu'elle comprend un amine carboxyborane répondant à la formule $R_1R_2NHBH_2C(O)OH$, dans laquelle $R_1$ et $R_2$ sont choisis parmi l'hydrogène, le méthyle, l'éthyle, le propyle et le n-butyle.

10. Composition pharmaceutique suivant la revendication 9, caractérisé en ce que $R_1$ et $R_2$ sont l'hydrogéne.

11. Composition pharmaceutique suivant la revendication 9, caractérisée en ce que $R_1$ et $R_2$ sont des méthyles.

12. Composition pharmaceutique suivant la revendication 9, caractérisée en ce que $R_1$ est le méthyle et $R_2$ est l'hydrogène.

13. Composition pharmaceutique ayant une activité anti-hyperlipémiante, caractérisée en ce qu'elle comprend un composé répondant à la formule $R_1$ $R_2$ $NHBH_2C(O)OH$ dans laquelle $R_1$ et $R_2$ sont choisis parmi l'hydrogène, le méthyle, l'éthyle, le propyle et le n-butyle.

14. Composition pharmaceutique suivant la revendication 13, caractérisé en ce que $R_1$ et $R_2$ sont l'hydrogène.

15. Composition pharmaceutique suivant la revendication 13, caractérisée en ce que $R_1$ et $R_2$ sont des méthyles.

16. Composition pharmaceutique suivant la revendication 13, caractérisée en ce que $R_1$ est le méthyle et $R_2$ est l'hydrogéne.

**Patentansprüche**

1. Verbindungen mit der Formel $R_1R_2NHBH_2C(O)OH$, worin $R_1$ und $R_2$ aus der Gruppe Wasserstoff, Methyl, Äthyl, Propyl und n-Butyl ausgewählt sind.

2. Verbindung nach Anspruch 1, dadurch gekennzeichnet, daß $R_1$ und $R_2$ Wasserstoff sind.

3. Verbindung nach Anspruch 1, dadurch gekennzeichnet, daß $R_1$ und $R_2$ Methyl sind.

4. Verbindung nach Anspruch 1, dadurch gekennzeichnet, daß $R_1$ Wasserstoff und $R_2$ Methyl ist.

5. Verfahren zur Herstellung der Verbindungen von Anspruch 1, dadurch gekennzeichnet, daß ein Teil Trimethylamin-Carboxyboran mit 10 Teilen einer Verbindung mit der Formel $R_1R_2NH$, worin $R_1$ und $R_2$ die im Anspruch 1 angegebenen Bedeutungen besitzen, bei einer Temperatur im Bereich von 20 bis 30°C für eine Zeitdauer von 15 Tagen umgesetzt wird.

6. Verfahren nach Anspruch 5, dadurch gekennzeichnet, daß $R_1$ und $R_2$ Wasserstoff sind.

7. Verfahren nach Anspruch 5, dadurch gekennzeichnet, daß $R_1$ und $R_2$ Methyl sind.

8. Verfahren nach Anspruch 5, dadurch gekennzeichnet, daß $R_1$ Wasserstoff und $R_2$ Methyl ist.

9. Pharmazeutische Zusammensetzung zur Verwendung bei der chemotherapeutischen Behandlung von Krebsgewebe dadurch gekennzeichnet, daß sie ein Amin-Carboxyboran mit der Formel $R_1R_2NHBH_2C(O)OH$ enthält, worin $R_1$ und $R_2$ aus der Gruppe Wasserstoff, Methyl, Äthyl, Propyl und n-Butyl ausgewählt sind.

10. Pharmazeutische Zusammensetzung nach Anspruch 9, dadurch gekennzeichnet, daß $R_1$ und $R_2$ Wasserstoff sind.

11. Pharmazeutische Zusammensetzung nach Anspruch 9, dadurch gekennzeichnet, daß $R_1$ und $R_2$ Methyl sind.

12. Pharmazeutische Zusammensetzung von Anspruch 9, dadurch gekennzeichnet, daß $R_1$ Methyl und $R_2$ Wasserstoff ist.

13. Pharmazeutische Zusammensetzung mit einer antihyperlipidämischen Wirksamkeit, dadurch gekennzeichnet, daß sie eine Verbindung mit der Formel $R_1R_2NHBH_2(O)OH$ enthält, worin $R_1$ und $R_2$ aus der Gruppe Wasserstoff, Methyl, Äthyl, Propyl und n-Butyl ausgewählt sind.

14. Pharmazeutische Zusammensetzung nach Anspruch 13, dadurch gekennzeichnet, daß $R_1$ und $R_2$ Wasserstoff sind.

15. Pharmazeutische Zusammensetzung nach Anspruch 13, dadurch gekennzeichnet, daß $R_1$ und $R_2$ Methyl sind.

16. Pharmazeutische Zusammensetzung nach Anspruch 13, dadurch gekennzeichnet, daß $R_1$ Methyl und $R_2$ Wasserstoff ist.